(19) Europäisches Patentamt
European Patent Office
Office européen des brevets

(11) EP 2 191 219 B1

(12) EUROPEAN PATENT SPECIFICATION

(45) Date of publication and mention
of the grant of the patent:
23.06.2021 Bulletin 2021/25

(51) Int Cl.:
F25J 3/00 (2006.01)          F25J 3/08 (2006.01)
A61L 2/02 (2006.01)          F25J 1/00 (2006.01)
F25J 1/02 (2006.01)

(21) Application number: 08828792.5

(22) Date of filing: 29.08.2008

(86) International application number:
PCT/US2008/074727

(87) International publication number:
WO 2009/029749 (05.03.2009 Gazette 2009/10)

(54) **METHODS FOR PRODUCING STERILE CRYOGENIC FLUIDS**

VERFAHREN ZUR HERSTELLUNG STERILER KRYOGENER FLÜSSIGKEITEN

PROCÉDÉS POUR PRODUIRE DES FLUIDES CRYOGÉNIQUES STÉRILES

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **30.08.2007 US 966796 P**

(43) Date of publication of application:
**02.06.2010 Bulletin 2010/22**

(73) Proprietor: **LINDE GAS NORTH AMERICA LLC Bridgwater, NJ 08807 (US)**

(72) Inventors:
• **LEE, Ron, Clark**
  **Bloomsbury, NJ 08804 (US)**
• **CHINH, Beatrice**
  **Summit, NJ 07901 (US)**

(74) Representative: **Gellner, Bernd**
  **Linde GmbH**
  **Intellectual Property EMEA**
  **Dr.-Carl-von-Linde-Straße 6-14**
  **82049 Pullach (DE)**

(56) References cited:
  **EP-A1- 0 888 132     EP-A2- 0 872 250**
  **US-A- 5 737 926      US-A1- 2005 155 378**

• **None**

**Description**

BACKGROUND OF THE INVENTION

The present invention provides for a method for producing sterile cryogenic fluids, such as sterile liquid by utilizing a waste gas stream from a heat exchanger as the sterile cryogenic fluid feedstock.

Gases that come into direct contact with pharmaceuticals during the production process have to meet the same high international quality specifications as the drugs themselves under the ICH Q7A and Food and Drug Administration drug policy. Cryogenic liquid nitrogen is increasingly being used to enhance drug production in the pharmaceutical and biotechnology industry. If the liquid nitrogen is not sterile, it cannot be used as a direct contact coolant in the preparation of final preparation of pharmaceuticals products such as antibiotics or vaccines. In addition cryogenic storage facilities have also fallen under scrutiny concerning the sterility of Liquid Nitrogen.

Typically the method of sterilization of a warm gas is by filtration to remove micro-organisms or other particulates whose size are generally greater than 0.2 $\mu$m. Sterile cryogenic fluids which are typically liquid nitrogen have been produced by two techniques where the method of sterilization is typically filtration. The first method is direct liquid filtration, and the second method is to filter ambient temperature (warm) gas in a standard filtration unit, then liquefy the sterile (warm) gas to produce sterile cryogenic liquid. U.S. Pat. No. 4,620,962 teaches that liquid nitrogen is split into two paths. The first stream passes through two heat exchangers and a (warm) filter for the production of sterile liquid nitrogen. The second stream is de-pressurized (to produce a colder liquid) and used to re-liquefy the first stream in the second of the heat exchangers. The overall liquid nitrogen consumption is about two times the amount of sterile liquid nitrogen produced. A similar method is described in EP 0 872 250, disclosing a method comprising the features of the preamble of independent claim 1.

As such it can be seen that in earlier processes, the amount of sterile cryogenic liquid produced is less than the amount of cryogenic liquid fed to the filtration system. The present invention provides for the production of a sterile cryogenic fluid through warm filtration while significantly reducing the amount of cryogenic fluid consumed.

SUMMARY OF THE INVENTION

The present invention, as defined by the appended claims, provides for a method for producing sterile cryogenic fluids by feeding a gas to be sterilized to a filtration system, thereby producing a sterile warm gas; feeding the sterile warm gas to a heat exchanger where it will contact a second stream of liquid, thereby cooling and condensing the sterile warm gas to produce a sterile liquid, and withdrawing the sterile liquid, the improvement comprising directing the warm waste gas stream from the heat exchanger to a second filtration unit to produce a sterile waste gas stream and feeding the sterile waste gas stream to a second heat exchanger to produce sterile cryogenic fluid by contact with a stream of liquid.

The present invention further provides for an improved method for producing sterile cryogenic fluids by feeding a warm gas stream to be sterilized to a filtration system to produce a sterile warm gas stream and feeding the sterile warm gas stream to a heat exchanger thereby cooling and condensing the sterile warm gas stream to produce a sterile liquid, the improvement comprising feeding the warm waste gas stream from the heat exchanger to a second filtration system for producing sterile warm gas. The warm gas streams are generally at or near ambient temperatures of about - 40°C to +40°C.

Alternatively to the present invention a method is thinkable for producing a sterile cryogenic fluid comprising feeding a waste gas stream from a first heat exchanger to a filtration system thereby producing a warm sterile gas and feeding the warm sterile gas to a second heat exchanger wherein the warm sterile gas is reduced to cryogenic temperature.

In a further embodiment, not according to the invention, there is a method for producing a sterile cryogenic fluid comprising the steps:

a) feeding a waste gas stream from a first heat exchanger unit to a filtration unit, whereby the waste gas stream is sterilized;

b) feeding the sterilized waste gas stream to a second heat exchanger wherein the sterilized waste gas stream is reduced in temperature, thereby producing a sterile cryogenic fluid;

c) recovering the sterile cryogenic fluid; and

d) feeding a second waste gas stream from the second heat exchanger and feeding the second waste gas stream to a second filtration unit whereby the second waste gas stream is sterilized and feeding the second sterilized waste gas stream to a third heat exchanger.

The gas stream that is fed to the filtration unit is typically a cryogenic fluid having a normal boiling point below -50°C and can be selected from the group consisting of nitrogen, oxygen, air and argon. Mixtures of cryogenic fluids, such as oxygen/nitrogen mixtures, are suitable cryogenic fluids. It is recognized that "fluids" can be either a gas or liquid having a normal boiling point below -50°C.

The filtration system removes microorganisms and other particulates from said gas stream while the heat exchanger will use cryogenic fluids to provide heat exchange with the sterilized gas stream.

The waste gas stream that leaves the heat exchanger is a cryogenic fluid selected from the group consisting of nitrogen, oxygen, air and argon. The waste gas stream and the gas stream can be either the same type of cryogenic fluids or they may be different.

Alternative embodiments provide for using the warm waste gas stream from the liquefaction step of the sterilization process as the warm feed stream for a second sterilization process. The inventive process may be modularized and a third or any number of additional modules may be added recognizing the constraints of pressure control throughout the modules. For example, a sterilized waste gas stream from a third heat exchanger may be fed to a third filtration unit.

The present invention will allow for the use of liquid nitrogen in key areas in drug production where it was formerly prohibitive to use liquid nitrogen due to contamination vector issues for introducing micro-organisms. This invention will also improve the overall efficiency of the sterilization process, particularly when two or more filtration unit/heat exchanger systems are employed.

BRIEF DESCRIPTION OF THE DRAWINGS

Fig. 1 is a schematic diagram of a process for sterilizing a cryogenic fluid employing a filtration unit and a heat exchange unit.

Fig. 2 is a schematic diagram of the present invention for producing a sterile cryogenic fluid by directing the warm waste gas stream from a first module to the feed stream of a second module.

Fig. 3 is a schematic diagram of the present invention for producing a sterile cryogenic fluid showing the use of three modules.

DETAILED DESCRIPTION OF THE INVENTION

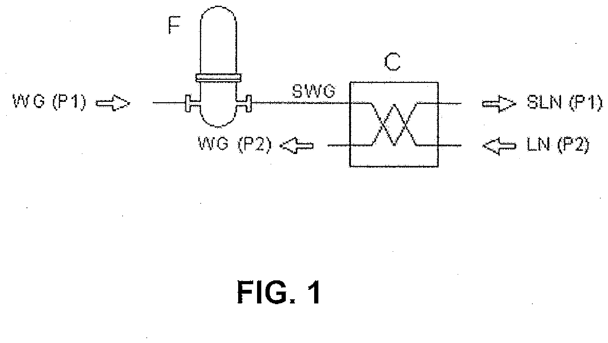

In Fig. 1, there is depicted a method for producing a sterile liquid fluid. For purposes of illustration, liquid nitrogen will be employed in the description but other gases that are capable of being sterilized may also be employed in the methods of the present invention.

Warm nitrogen gas WG at a pressure P1 is delivered through a first line and is filtered by filtration unit F to produce sterile warm gas SWG. The filtration process, using widely accepted filtration methods for gaseous nitrogen, removes microorganisms such as virus, bacteria and fungi. This sterile warm gas SWG directed through a second line and is then liquefied in a heat exchanger C, of conventional design appropriate for cryogenic operation using a separate stream of liquid nitrogen LN at a pressure P2 fed into the heat exchanger through a fourth line . Suitable heat exchangers include plate fin type compact heat exchangers.

P2 must be lower than P1 in order to satisfy the thermodynamic requirement for condensing the sterile warm gas. That requirement is that the saturation temperature at P1 must be higher by about 5°C than the saturation temperature at P2. This means that P2 will be about 1 to 3 bar (14.5 to 43.5 psi) lower in pressure depending on the value of P1. For example, if P1 is 10 barg, then P2 would need to be about 6.9 barg (3.1 bar pressure difference) for a 5°C temperature difference between the two corresponding saturation temperatures. If P1 is 3 barg, then P2 would need to be about 1.6 barg or 1.4 bar difference in pressure for the same 5°C temperature difference between the two corresponding saturation temperatures.

The sterile liquid nitrogen SLN will exit the heat exchanger C through a third line where it may be recovered and used or stored for later use. The warm waste gas stream WG at pressure P2 exits the heat exchanger C through a fifth line where it may be recovered for reuse elsewhere or, more typically, is by release into the atmosphere.

The ratio of liquid nitrogen LN used to the amount of sterile liquid nitrogen SLN produced in the system illustrated in Fig 1 is typically a number somewhat greater than 1. A ratio of about 1.1 is typical. For purposes of illustration a ratio of 1.0 will be used. In this case, the total nitrogen used is two times the amount of sterile liquid nitrogen produced. As such,

$$FOM = \frac{\text{Amount of nitrogen consumed}}{\text{Amount of sterile liquid nitrogen produced}} - 1$$

The solution FOM is a dimensionless measure of the excess fluid (warm or cryogenic) required to produce a given amount of sterile cryogenic liquid, and will typically range between 0 and 1. For the system shown in Fig. 1, the FOM is about 1.0. The lower the FOM, the more efficient the system.

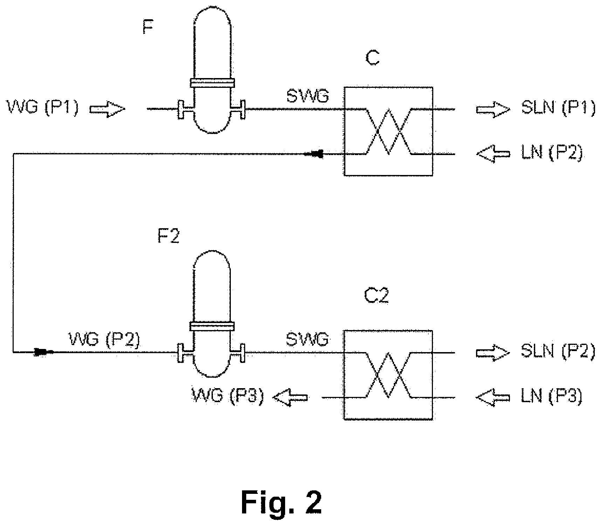

Fig. 2 describes the present invention for reducing the amount of nitrogen required in the process. Two modules of similar construction are connected as shown. The first module works as described above in Fig. 1. Warm gas WG at a pressure P1 is directed through a first line into an appropriate filtration unit F and sterile warm gas SWG is fed into a heat exchanger C through a second line where it will contact liquid nitrogen LN fed through a fourth line at a lower pressure P2 than the WG and a sterile liquid nitrogen SLN stream is produced. The SLN at P1 is recovered via a third line

The warm waste gas stream WG which is also at pressure P2 is fed through a fifth line to a second filtration unit F2 which produces the sterile warm gas SWG which is fed through a sixth line to a second heat exchanger C2 and produces the sterile liquid nitrogen SLN at pressure P2 which is then recovered through a seventh line. Liquid nitrogen LN at pressure P3 which is lower than P1 and P2 contacts the SWG through an eighth line and the warm waste gas WG exits the heat exchanger C2 through a ninth line at a pressure P3.

The pressure of the liquid nitrogen LN in each of the heat exchangers must be lower than the pressure of the corresponding sterile warm gas SWG. For this example, suitable pressures would be P1 = 9 barg, P2 = 6.1 barg and P3 = 3.9 barg. These pressure differentials ensure about 5°C temperature difference between the saturation temperatures in each of the heat exchangers.

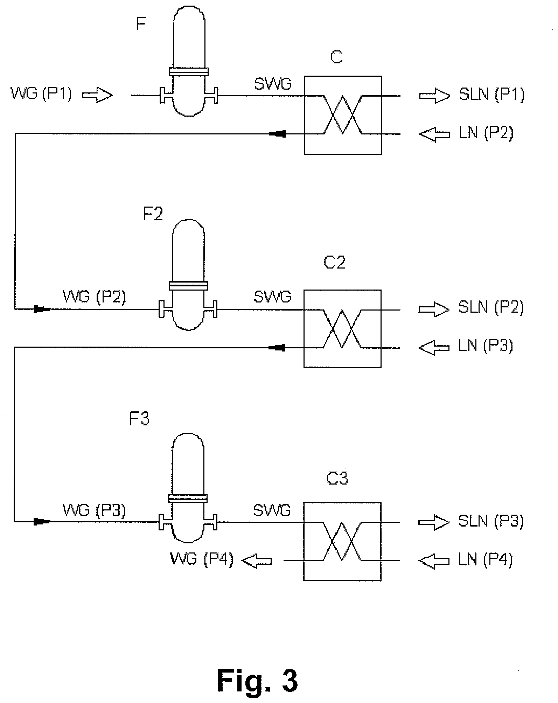

Fig. 3 describes a system where a third module may be added to that described in Fig. 2. The description of the operation of the first two modules is the same as that described above for Fig. 2. The warm waste gas WG that leaves the second heat exchanger C2 through a ninth line enters the filtration unit F3 at pressure P3. The sterile warm gas SWG enters the third heat exchanger C3 through a tenth line and contacts the liquid nitrogen LN introduced through a twelfth line at pressure P4 to produce the sterile liquid nitrogen SLN at pressure P3. The warm waste gas WG will exit the heat exchanger C3 at pressure P4 through a thirteenth line while the SLN at P3 will be recovered through an eleventh line. The three module system envisioned in Fig. 3 has an FOM of about 0.33. Suitable operating pressures here are P1 = 9 barg, P2 = 6.1 barg, P3 = 3.9 barg, and P4 = 2.2 barg.

While liquid nitrogen has been used as the cryogenic fluid for the above discussion, other cryogenic fluids, i.e., those having a normal boiling point below -50°C such as oxygen, liquid air and argon may be employed in the methods of the present invention.

The method for warm sterilization may be by filtration with filters having a pore size typically of about 0.2 micron or less but it may also be any combination of filtration with other treatment technologies.

The cryogenic and ambient temperature fluid sources may be from single or multiple storage tanks or processes. For example, a single pressured cryogenic liquid storage tank may be used to supply cryogenic liquid and warm vapor to the sterilization modules at various pressures and flow rates. The pressure regulation devices and flow control valves are not show but would be typical for the processes so described and known to those of ordinary skill in the art.

The individual stream flows may also be modified within the scope of the present invention. For example, additional vent streams may be added to improve flow control and balance the heat transfer requirements of the system. Provisions may be made to allow individual modules to operate without interaction at times or during start-up of the system.

Additional heaters or ambient vaporizers may be added to perform additional warming or vaporization of the cryogenic fluids. For example, the outlet gas (WG) from the heat exchangers shown in Figs. 1 to 3 may still be too cold for use in subsequent process steps, e.g., filtration. The initial input of warm gas to the first module will typically come from a cryogenic liquid storage tank and will need to be vaporized and warmed.

Additional components may be added to the system to accomplish steam sterilization or similar processes in order to prepare the system for operation.

The clean but not sterile vent (waste) gas (WG) from the process may also be used for other purposes. For example, warm, pressurized nitrogen gas may be used for a variety of purposes such as tank inerting and pressure transfer. Some or all of this vent gas may be fed directly into a common gas use header within a process facility for general use.

**Claims**

1. A method for producing sterile cryogenic fluids (SLN (P1)) by feeding a gas stream (WG(P1)) to a filtration system (F) to produce a sterile warm gas (SWG) and feeding said sterile warm gas (SWG) to a heat exchanger (C) where it will contact a second stream of liquid (LN (P2)), thereby cooling and condensing said sterile warm gas (SWG) to produce a sterile liquid (SLN (P1)) and a warm waste gas stream (WG (P2)), and withdrawing the sterile liquid (SLN (P1)), **characterized by** the method further comprising, feeding the warm waste gas stream (WG(P2)) from the condensing step in said heat exchanger (C) to a second filtration system (F2) for producing a sterile waste gas stream (SWG) and feeding the sterile waste gas stream (SWG) from said second filtration system (F2) to a second heat exchanger (C2) to produce sterile cryogenic fluid (SLN(P2)) by contact with a stream of liquid (LN (P3)).

2. The method as claimed in claim 1 wherein said gas stream is selected from a group consisting of a cryogenic fluid having a normal boiling point below -50°C.

3. The method as claimed in claim 2 wherein said cryogenic fluid is selected from a group consisting of nitrogen, oxygen, air and argon, and mixtures thereof.

4. The method as claimed in any of claims 1 to 3 wherein said first and said second filtration system (F) removes microorganisms and other particulates from said gas stream (WG(P1)), respectively from said warm waste gas stream (SWG).

5. The method as claimed in any of claims 1 to 4 wherein said heat exchanger (C) employs cryogenic fluids to provide heat exchange.

6. The method as claimed claim 5 wherein the pressure of said cryogenic fluids to provide heat exchange is less than the pressure of said gas stream.

7. The method as claimed in any of claims 1 to 6 wherein said sterile cryogenic fluids are recovered.

8. The method as claimed in any of claims 1 to 7 wherein said waste gas stream is a cryogenic fluid selected from a group consisting of nitrogen, oxygen, air and argon.

9. The method as claimed in any of claims 1 to 8 wherein said gas stream and said waste gas stream are both cryogenic fluids.

**Patentansprüche**

1. Verfahren zum Herstellen steriler kryogener Flüssigkeiten (SLN (P1)) durch Zuführen eines Gasstroms (WG(P1)) zu einem Filtrationssystem (F), um ein steriles warmes Gas (SWG) herzustellen, und Zuführen des sterilen warmen Gases (SWG) zu einem Wärmetauscher (C), wo es mit einem zweiten Flüssigkeitsstrom (LN (P2)) in Kontakt kommt, wodurch das sterile warme Gas (SWG) gekühlt und kondensiert wird, um eine sterile Flüssigkeit (SLN (P1)) und einen warmen Abgasstrom (WG (P2)) herzustellen, und Abziehen der sterilen Flüssigkeit (SLN (P1)), **gekennzeichnet durch** das Verfahren ferner umfassend das Zuführen des warmen Abgasstroms (WG (P2)) aus dem Kondensationsschritt in dem Wärmetauscher (C) zu einem zweiten Filtrationssystem (F2) zum Herstellen eines sterilen Abgasstroms (SWG) und Zuführen des sterilen Abgasstroms (SWG) aus dem zweiten Filtrationssystem (F2) zu einem zweiten Wärmetauscher (C2), um sterile kryogene Flüssigkeit (SLN(P2)) durch Kontakt mit einem Flüssigkeitsstrom (LN (P3)) herzustellen.

2. Verfahren nach Anspruch 1, wobei der Gasstrom ausgewählt ist aus einer Gruppe bestehend aus einer kryogenen Flüssigkeit mit einem normalen Siedepunkt unter -50 °C.

3. Verfahren nach Anspruch 2, wobei die kryogene Flüssigkeit ausgewählt ist aus einer Gruppe bestehend aus Stickstoff, Sauerstoff, Luft und Argon und Gemischen davon.

4. Verfahren nach einem der Ansprüche 1 bis 3, wobei das erste und das zweite Filtrationssystem (F) Mikroorganismen und andere Partikel aus dem Gasstrom (WG (P1)) bzw. aus dem warmen Abgasstrom (SWG) entfernt.

5. Verfahren nach einem der Ansprüche 1 bis 4, wobei der Wärmetauscher (C) kryogene Flüssigkeiten verwendet, um einen Wärmeaustausch bereitzustellen.

6. Verfahren nach Anspruch 5, wobei der Druck der kryogenen Flüssigkeiten, um einen Wärmeaustausch bereitzustellen, geringer ist als der Druck des Gasstroms.

**7.** Verfahren nach einem der Ansprüche 1 bis 6, wobei die sterilen kryogenen Flüssigkeiten gewonnen werden.

**8.** Verfahren nach einem der Ansprüche 1 bis 7, wobei der Abgasstrom eine kryogene Flüssigkeit ist, ausgewählt aus einer Gruppe bestehend aus Stickstoff, Sauerstoff, Luft und Argon.

**9.** Verfahren nach einem der Ansprüche 1 bis 8, wobei der Gasstrom und der Abgasstrom beide kryogene Flüssigkeiten sind.

## Revendications

**1.** Procédé pour produire des fluides cryogéniques stériles (SLN (P1)) en alimentant un flux de gaz (WG(P1)) dans un système de filtration (F) pour produire un gaz chaud stérile (SWG) et en alimentant ledit gaz chaud stérile (SWG) dans un échangeur thermique (C) où il sera en contact avec un deuxième flux de liquide (LN (P2)), refroidissant et condensant de ce fait ledit gaz chaud stérile (SWG) pour produire un liquide stérile (SLN (P1)) et un flux de gaz résiduaire chaud (WG (P2)), et en retirant le liquide stérile (SLN (P1)), **caractérisé en ce que** le procédé comprend en outre, l'alimentation du flux de gaz résiduaire chaud (WG(P2)) provenant de l'étape de condensation dans ledit échangeur thermique (C) dans un deuxième système de filtration (F2) pour produire un flux de gaz résiduaire stérile (SWG) et l'alimentation du flux de gaz résiduaire stérile (SWG) provenant dudit deuxième système de filtration (F2) dans un deuxième échangeur thermique (C2) pour produire un fluide cryogénique stérile (SLN(P2)) par contact avec un flux de liquide (LN (P3)).

**2.** Procédé selon la revendication 1, dans lequel ledit flux de gaz est choisi parmi un groupe constitué d'un fluide cryogénique ayant un point d'ébullition normal inférieur à -50 °C.

**3.** Procédé selon la revendication 2, dans lequel ledit fluide cryogénique est choisi parmi un groupe constitué d'azote, d'oxygène, d'air et d'argon, et mélanges de ceux-ci.

**4.** Procédé selon l'une quelconque des revendications 1 à 3, dans lequel ledit premier et ledit deuxième système de filtration (F) éliminent des microorganismes et d'autres particules dudit flux de gaz (WG(P1)), respectivement dudit flux de gaz résiduaire chaud (SWG).

**5.** Procédé selon l'une quelconque des revendications 1 à 4, dans lequel ledit échangeur thermique (C) emploie des fluides cryogéniques pour fournir un échange thermique.

**6.** Procédé selon la revendication 5, dans lequel la pression desdits fluides cryogéniques pour fournir un échange thermique est inférieure à la pression dudit flux de gaz.

**7.** Procédé selon l'une quelconque des revendications 1 à 6, dans lequel lesdits fluides cryogéniques stériles sont récupérés.

**8.** Procédé selon l'une quelconque des revendications 1 à 7, dans lequel ledit flux de gaz résiduaire est un fluide cryogénique choisi parmi un groupe constitué d'azote, d'oxygène, d'air et d'argon.

**9.** Procédé selon l'une quelconque des revendications 1 à 8, dans lequel ledit flux de gaz et ledit flux de gaz résiduaire sont tous deux des fluides cryogéniques.

**FIG. 1**

**Fig. 2**

**Fig. 3**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4620962 A

- EP 0872250 A